(19) Europäisches Patentamt
European Patent Office
Office européen des brevets

(11) EP 1 985 310 B1

(12) **EUROPEAN PATENT SPECIFICATION**

(45) Date of publication and mention of the grant of the patent:
**20.03.2013 Bulletin 2013/12**

(51) Int Cl.:
***A61K 47/36*** *(2006.01)* ***A61P 15/10*** *(2006.01)*
***A61K 9/26*** *(2006.01)* ***A61K 31/4985*** *(2006.01)*

(21) Application number: **08251529.7**

(22) Date of filing: **25.04.2008**

(54) **Solid dosage forms**

Feste Dosierformen

Formes de dosage solide

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **25.04.2007 US 926403 P**
**22.05.2007 US 931449 P**

(43) Date of publication of application:
**29.10.2008 Bulletin 2008/44**

(73) Proprietor: **Teva Pharmaceutical Industries Ltd.**
**49131 Petah Tiqva (IL)**

(72) Inventors:
- **Zalit, Ilan**
**Rosh Ha Ayin (IL)**
- **Triger-Messer, Yonit**
**Tel-Mond (IL)**

(74) Representative: **Wong, Kit Yee et al**
**D Young & Co LLP**
**120 Holborn**
**London EC1N 2DY (GB)**

(56) References cited:
**WO-A-2005/000296** **US-A1- 2003 235 617**
**US-A1- 2006 099 252** **US-A1- 2006 276 442**
**US-B1- 6 821 975**

- **R. ROWE ET AL, ED.: "handbook of pharmaceutical excipients, ed.4" 2003, AMERICAN PHARMACEUTICAL ASSOCIATION 277990 , XP002489155 * page 603 - page 611 ***
- **DATABASE CA CHEMICAL ABSTRACTS SERVICE, COLUMBUS, OHIO, US; XP002489156 retrieved from STN Database accession no. 144: 398306 & CN 1 742 732 A (XI CHEN ET AL.) 8 March 2006 (2006-03-08)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

FIELD OF THE INVENTION

This invention relates to compressed solid dosage forms, such as tablets and caplets, and manufacturing methods thereof. The invention relates more particularly to tabletting manufacturing methods and tablets produced therefrom for drugs of low aqueous solubility such as tadalafil.

The present invention is defined in the appended claims. Subject-matter which is not encompassed by the scope of the claims does not form part of the present invention.

BACKGROUND OF THE INVENTION

When solid dosage forms are taken orally, in many cases the drug must dissolve in aqueous gastrointestinal fluids in, e.g., the patient's stomach, before the drug can exert a therapeutic effect. A recurring problem with compressed solid oral dosage forms, such as tablets and caplets (i.e., capsule-shaped tablets) is that the rate of dissolution of some drugs from the dosage form limits their biological availability. This problem is especially common for drugs that are small organic molecules with low solubility in aqueous fluids.

There are several ways to address the solubility issue of poorly soluble drugs. For example, the drug itself can be modified. The physical form of the drug can be manipulated by various techniques to optimize the rate at which the drug dissolves. Of these techniques, the one most relevant to the present invention is particle size reduction. The rate of dissolution of a solid may often depend upon the surface area that is exposed to the dissolving medium; and because the surface area of a given mass of a substance is generally inversely proportional to the substance's particle size, reducing the particle size of a powder or granular substance may increase its dissolution rate.

When it is effective, particle size reduction increases the dissolution rate of a particulate solid by increasing the surface area that is exposed to the dissolving medium However, particle size reduction is not always effective in increasing the dissolution rate of a drug in a compressed solid dosage form. During the dosage form manufacturing process, many hydrophobic drugs have a strong tendency to agglomerate into larger particles, resulting in an overall decrease in effective surface area. Remington (The Science and Practice of Pharmacy, 20th ed. 656, 657, A.R. Gennaro Ed., Lippincott Williams & Wilkins, Philadelphia 2000) contains a more thorough discussion of the concept of "effective surface area" and the effect of particle size on dissolution. A drug that has ostensibly been milled to a fine particle size will sometimes display dissolution characteristics of those with larger particle size due to agglomeration or similar effect.

There are three well-known processes for manufacturing compressed solid dosage forms: the wet granulation method, the double-compression method (also known as dry granulation), and the direct compression method. In each of these methods, there are blending steps that can promote agglomeration of fine particles of the drug into larger, less rapidly dissolving particles.

In the wet granulation method, pre-weighed drug and one or more other ingredients, such as a diluent, are blended. The blend is then mixed with a liquid such as water or ethanol, which causes the particles to agglomerate into a damp mass. Optionally, the liquid contains a binder. The damp mass is screened to produce granules that are subsequently dried. The dry granules are screened to produce granules of a predetermined size. Then, the granules are typically blended with a solid lubricant and optionally other ingredients. Lastly, the lubricated granules and any other extra-granular ingredients are compressed into a tablet, which may subsequently be coated.

The double-compression or dry granulation method has fewer steps than wet granulation and does not require contact with a liquid or drying, which makes the method well suited for formulating water-sensitive and heat-sensitive drugs. In the double-compression method, the drug and other ingredients, such as a lubricant, are blended and then compressed in a first compression step. There are two conventional first compression techniques. One is roller compaction, in which the blend is fed between rollers, which press the blend into sheets; the other is slugging, in which the blend is compressed into slugs, which are tablet-like forms that are typically larger than tablets intended for human consumption. The resulting sheets or slugs are then comminuted into granules, mixed with a solid lubricant, and compressed in a second compression step to produce the final tablet.

The direct compression method is the simplest of the three well-known methods for making compressed solid dosage forms. In the direct compression method, the drug and any other ingredients are blended together and directly compressed into the final tablet. The tablet ingredients must have good flow properties and cohesion to be suitable for direct compression tabletting. Microcrystalline cellulose and lactose are two commonly used diluents in direct compression tabletting.

In the development and manufacturing of formulations of poorly water-soluble drugs, when high bioavailability of the drug is required, usually a combination of small drug particles (such as micronized drug particles) and a suitable manufacturing process (for example, one of the methods described above) is used. The micronization process, however, can involve high costs in time and process efficiency, and may also present a safety issue, since the micronization

process may result in fine drug powder.

Accordingly, any new composition and/or manufacturing process that will allow desirable dissolution and bioavailability rates while using relatively large drug particles can allow safer and more economic processes for the manufacture of solid dosage forms.

Tadalafil, the active ingredient in Cialis®, has been used for the treatment of male erectile dysfunction. The prescribing information for Cialis® describes this product as film-coated, almond-shaped tablets for oral administration, containing tadalafil and the following inactive ingredients: croscarmellose sodium, hydroxypropyl cellulose, hypromellose, iron oxide, lactose monohydrate, magnesium stearate, microcrystalline cellulose, sodium lauryl sulfate, talc, titanium dioxide, and triacetin (see http://pi.lilly.com/us/cialis-pi.pdf).

Tadalafil has the chemical name (6R-trans)-6-(1,3-benzodioxol-5-yl)-2,3,6,7,12,12a-hexahydro-2-methyl-pyrazino[1',2':1,6]pyrido[3,4-b]indole-1,4-dione. The structure of tadalafil (CAS# 171596-29-5) is shown below:

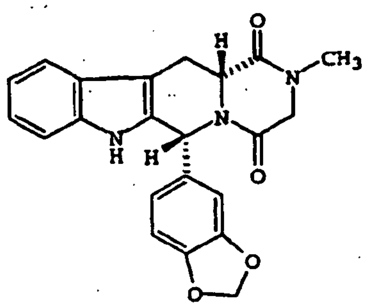

Tadalafil is a solid that is understood to be practically insoluble in water and only very slightly soluble in some organic solvents, such as methanol, ethanol, and acetone. United States Patent No. 6,841,167 reports that tadalafil has a water solubility of about 2 μg per mL of water at 25°C.

Different techniques have been applied in an attempt to overcome the apparent poor water solubility of tadalafil. US 6,841,167 reports a pharmaceutical formulation comprising tadalafil in "free drug" form in admixture with a diluent, lubricant, a hydrophilic binder, and a disintegrant. Soft capsules containing tadalafil suspended in a pharmaceutically acceptable solvent have purportedly developed in an attempt to prepare formulations of tadalafil with supposedly improved bioavailability.

Another technique applied to improve solubility is described in US Patent No. 5,985,326 and involves preparing formulations using "co-precipitates" of tadalafil, wherein an "intimate mixture" of tadalafil and a carrier in a non-aqueous water miscible solvent, and, optionally, water are co-precipitated from the "intimate mixture" using an aqueous "co-precipitation medium" in which the carrier is substantially insoluble.

U.S. Patent No. 6,821,975 is listed in the United States Federal Food and Drug Administration ("FDA")'s "Orange Book" for the Cialis® product, and is assigned on its face to the company that markets Cialis®. This patent is purportedly directed to a "free drug particulate form" of tadalafil "comprising particles of the compound wherein at least 90% of the particles have a particle size of less than about 40 microns." Preferably at least 90% of the particles have a particle size of less than 10 microns.

The above-described methods for increasing the bioavailability of tadalafil suffer from economical and safety disadvantages. US 6,821,975 requires micronization, which can be time-consuming and also could raise safety issues due to the fine powder produced therefrom. US 5,985,326 requires the use of significant amounts of organic solvent, which is environmentally undesirable. Accordingly, a method which uses large drug particles (with $d_{(0.9)}$ of 40 microns or more) while maintaining desirable bioavailability will improve the economy and safety of the manufacturing process of tadalafil tablets.

Levy *et al.* reported on the effect of starch on the rate of dissolution of salicylic acid from tablets manufactured by double compression (Levy, G. et al, J. Pharm. Sci. 1963, 52, 1047). It was reported that increasing the starch content from 5 to 20% increased the rate of dissolution of salicylic acid three-fold. This observation was attributed to faster disintegration of tablets with a higher starch content. In 1966, Finholt *et al.* observed that fine starch particles added to phenobarbital tablets increased the dissolution rate of phenobarbital from the tablets. Reaching a different conclusion from Levy *et al.*, Finholt *et al.* proposed that the starch worked by coating the phenobarbital crystals and imparting a hydrophilic property to them, which improved contact between the phenobarbital particles and an aqueous dissolution medium (Finholt, P. Medd Norsk Farm. Selsk. 1966, 28, 238).

Starch is a common ingredient of tablets, where it is used for a variety of purposes. It is routinely used, for example, as a diluent, binder, disintegrant, and glidant. Diluents increase the bulk of a solid pharmaceutical composition and can make a pharmaceutical dosage form containing the composition easier for the patient and caregiver to handle. Binders help bind the active ingredient and other ingredients together, for example, during granulation or compression

steps. Disintegrants accelerate break up of the tablet in a patient's stomach, typically by drawing water into the tablet and causing it to swell, thereby breaking the tablet into smaller pieces (resulting in greater surface area). Glidants improve the flowability of powder compositions by coating the surfaces of the particles. According to the Handbook of Pharmaceutical Excipients (4th Ed. 603-604; Pharmaceutical Press: London 2003) starch is commonly used in an amount of 5-15% when it functions as a binder. When functioning as a disintegrant, starch is commonly added in an amount of 3-15%. The amount of diluent that is called for in a particular application depends upon many parameters and is highly variable. However, as the Handbook notes, starch does not compress well and tends to increase tablet friability and capping if used in high concentrations. Thus, the use of high concentrations of starch as a diluent is often limited by the deterioration in the hardness and friability (resistance to chipping) that occurs as the proportion of starch in the formulation is increased.

In US Publication No. 2006/0099252, a pharmaceutical formulation and a method of manufacturing thereof is disclosed. The publication discloses formulations containing high amounts of starch, and methods of obtaining such formulations, said methods comprising blending the active compound with starch, compressing the blend into a solid, comminuting the solid into granules, wetting and drying the granules, and tabletting the dried granules to a solid formulation. As a result of this process a significant increase in dissolution rate of low water soluble drugs was observed.

WO 2005/000296 discloses an orally deliverable pharmaceutical composition comprising a drug with low water solubility and pregelatinized starch having low viscosity and/or exhibiting a multimodal particle size distribution. The formulation disclosed in WO 2005/000296 is reported to exhibit increased drug dissolution rate consistency.

In US Publication No. 2008/0009502, a solid composite and a method of making thereof is disclosed. The publication discloses solid composite comprising tadalafil and at least one carrier. Preferably, the carrier is a hydrophilic polymer such as povidone, hydroxypropyl methylcellulose, and polyethylene glycol.

It would be highly desirable to produce a compressed solid dosage form for oral administration having a high rate of dissolution of a poorly soluble drug with minimal reduction of the particle size of the drug.

The present invention therefore seeks to provide improved pharmaceutical dosage forms for tadalafil which display high dissolution rates and which avoid the need to reduce the particle size of the drug (for example, by micronization).

## SUMMARY OF THE INVENTION

The invention provides a compressed solid pharmaceutical dosage form comprising tadalafil, pregelatinised starch, and a binder, wherein the tadalafil has a particle size distribution such that $d_{(0.9)}$ , as measured by laser diffraction, is greater than or equal to 40 μm, wherein the weight ratio of starch to tadalafil is S:1 or more, 6:1 or more, 10:1 or more, 12:1 or more, 15:1 or more, and is 100:1 to less.

The pharmaceutical dosage form comprises a binder. Preferably, the starch to binder weight ratio in the solid dosage form is between about 1:1 and about 9:1.

In one aspect, the pharmaceutical dosage form displays a high rate of dissolution. For example, about 60% or more, or about 68% or more, of the tadalafil is released within 20 minutes when a sample containing 20 mg of tadalafil is tested in a medium at least as stringent as an 1000 mL aqueous medium containing 0.14 wt % sodium lauryl sulfate and 6 g/L $NaH_2PO_4$ and having a pH of 4.5 at 37°C, using USP Apparatus II with paddles rotating at a speed of 50 rpm. Preferably, about 65% or more, more preferably about 76% or more, of the tadalafil is released within 40 minutes under the same condition.

In a one aspect, the pharmaceutical dosage form displays similar dissolution rate as Cialis® of the same dosage at the same time point under the same testing condition. Preferably, the ratio of dissolution rate at the same time point under the same testing condition between the dosage form and Cialis® of the same dosage is between 80% and 125%.

In one aspect, the pharmaceutical dosage form is bioequivalent to Cialis® of the same dosage. Preferably, the $C_{max}$ ratio of the dosage form to Cialis® of the same dosage is between 80% and 125%. Preferably, the AUC ratio of the dosage form to Cialis® of the same dosage is between 80% and 125%.

One embodiment of the invention provides a process for preparing the above-described solid pharmaceutical dosage form comprising tadalafil, pregelatinised starch, and a binder as further defined in the claims.

One embodiment of the invention provides a process for preparing a solid dosage form of tadalafil as defined in the claims by using wet granulation or wet double compression method.

One embodiment of the invention provides a process for preparing the solid dosage form of tadalafil as defined in the claims, comprising:

a) blending particulate tadalafil with starck;
b) wet-granulating the product of step a) with a granulating liquid; and
c) drying the product of step b) to form dried granules.

The process further comprises compressing the granules from the last step to form a compressed solid dosage form.

One embodiment of the invention provides a solid pharmaceutical dosage form of the invention for use in medicine, preferably for use in treating male erectile dysfunction.

One embodiment of the invention provides the use of a solid pharmaceutical dosage form of the invention in the preparation of a medicament for treating male erectile dysfunction.

BRIEF DESCRIPTION OF THE DRAWINGS

Figure 1 is a plot comparing the dissolution rates of tadalafil over time for:

(1) (a) a formulation containing tadalafil mixed with starch;

(b) a formulation containing tadalafil mixed with starch and pressed to slugs;
(c) a formulation containing tadalafil mixed with starch, pressed to slugs, and milled;
(d) a formulation containing tadalafil mixed with starch, pressed to slugs, milled, and wet granulated;

(2) Tadalafil Formulation 2, containing conventional fillers and no starch;
(3) Tadalafil Formulation 3, containing starch and prepared according to Example 3;
(4) Tadalafil Formulation 4, containing low amounts of starch and PVP, and prepared according to Example 4;
(5) Tadalafil Formulation 5, containing starch and surfactant, and prepared according to Example 5;
(6) Tadalafil Formulation 7, containing starch and prepared according to Example 7.
(7) Tadalafil Formulation 8, prepared according to Example 8, which follows FORMULATION 5 of US 2006/099252, except that the raloxifene in FORMULATION 5 is replaced with tadalafil.
(8) Cialis®, 20mg.

DETAILED DESCRIPTION OF THE INVENTION

As used herein, the term "PVP" or "povidone" refers to polyvinylpyrrolidinone, the term "hypromellose" refers to hydroxypropyl methylcellulose, the term meglumine refers to N-methyl-D-glucamine, and the term "Tris" refers to tris (hydroxymethyl)amine.

As used herein, the term "invert sugar" refers to an equimolar mixture of two monosaccharides, glucose and fructose. Invert sugar may be produced by hydrolysis of sucrose.

As used herein, the term "$d_{(0.9)}$" refers to the 90th percentile of the particle size distribution. The $d_{(0.9)}$ is a value on the distribution that 90% of the particles (by accumulative volume) have a size of this value or less, when the particle size distribution is measured by conventionally accepted method such as laser diffraction. Instruments for measuring particle size distribution can be obtained from various sources, such as from Malvern Instruments Ltd. (U.K.).

As used herein, unless otherwise specified, all percentages are percentage by weight based on the total weight of the solid dosage form.

As used herein, the product referred to as Cialis® is exemplified by Lot No. A149562.

As used herein, dissolution rate is measured by the percentage of tadalafil dissolved in a liquid medium. The testing condition is as follows: a sample containing 20 mg of tadalafil is tested in an 1000 mL aqueous medium containing 0.14 wt % sodium lauryl sulfate and 6 g/L $NaH_2PO_4$ and having a pH of 4.5 at 37°C, using USP Apparatus II with paddles rotating at a speed of 50 rpm.

As used herein, "similar dissolution rate" to Cialis® refers to the ratio of dissolution rate at the same time point under the same testing condition between the dosage form and Cialis® of the same dosage is between 80% and 125%.

As used herein, "bioequivalence" or "bioequivalent" means that the product meets or would meet the FDA's requirement for bioequivalence. According to the United States Code of Federal Regulation, Title 21, Vol. 5, § 320.1, "Bioequivalence means the absence of a significant difference in the rate and extent to which the active ingredient or active moiety in pharmaceutical equivalents or pharmaceutical alternatives becomes available at the site of drug action when administered at the same molar dose under similar conditions in an appropriately designed study." For example, one criterion relied on by the FDA is defined in the FDA's Statistical Procedures for Bioequivalence Studies Using a Standard Two-Treatment Crossover Design (1992), which is incorporated herein by reference. Bioequivalence to Cialis® refers to the 90% confidence interval of the $C_{max}$ ratio and/or of the $AUC_{max}$ ratio being between 80% and 125%.

As used herein, for tadalafil, the term "$C_{max}$" refers to maximum plasma concentration of tadalafil following the ingestion of a dose of tadalafil; the term "$T_{max}$" refers to the time following ingestion when $C_{max}$ is reached; the term "$AUC_{0-t}$" refers to the area under curve of plasma concentration of tadalafil versus time from ingestion. The curve usually ends when the concentration is below the detection limit. The term "$AUC_{0-t}$" refers to the AUC at time t from ingestion. $AUC_{0-\infty}$ is the total area under the curve and is a measure of the patient's total exposure to tadalafil. When a study

involves multiple subjects, $C_{max}$ refers to the geometric mean of the $C_{max}$ of multiple subjects, and AUC refers to the geometric mean of the AUC of multiple subjects in the study.

As used herein, the term "$C_{max}$ ratio" refers to the ratio of the $C_{max}$ of a test dosage form to the $C_{max}$ of the reference product Cialis® of the same dosage. The $C_{max}$ ratio is defined as

$$C_{max}\ \text{ratio} = C_{max(test)}/C_{max\ (ref)},$$

As used herein, the term "AUC ratio" refers to the ratio of the AUC of a test product to the corresponding AUC of the reference product Cialis® of the same dosage. The AUC ratio is defined as

$$\text{AUC ratio} = AUC_{(test)}/AUC_{(ref)},$$

As used herein, "fed state" refers to the state of subject who has eaten an FDA-recommended high fat (approximately 50 percent of total caloric content of the meal) and high-calorie (approximately 800 to 1000 calories) meal or equivalent. Preferably, this test meal should derive approximately 150, 250, and 500-600 calories from protein, carbohydrate, and fat, respectively, as set forth in FDA's Guidance for Industry: Food-Effect Bioavailability and Fed Bioequivalence Studies (1992), which is incorporated herein by reference.

As used herein, "fasted state" refers to the state of subject who has not eaten for at least ten hours, typically overnight, prior to ingestion of the dosage form. Preferably, no food should be allowed for at least 4 hours post-dose, as set forth in FDA's Guidance for Industry: Food-Effect Bioavailability and Fed Bioequivalence Studies (1992).

One embodiment of the invention provides a solid pharmaceutical dosage form comprising tadalafil and starch, wherein the tadalafil has a particle size distribution such that $d_{(0.9)}$ is greater than or equal to 40 $\mu$m, wherein the weight ratio of starch to tadalafil is about 4.5:1 or more.

Another embodiment of the invention provides a solid pharmaceutical dosage form comprising tadalafil and starch, wherein the tadalafil has a particle size distribution such that $d_{(0.9)}$ is greater than or equal to 40 $\mu$m, wherein the amount of starch in the solid pharmaceutical dosage form is between about 45% and about 60% by weight.

In a preferred embodiment of the invention, the tadalafil has a particle size distribution such that $d_{(0.9)}$ is greater than or equal to about 50 $\mu$m, greater than or equal to about 60, or greater than or equal to about 70 $\mu$m. Preferably, the tadalafil has a particle size distribution such that $d_{(0.9)}$ is less than or equal to about 150 $\mu$m or less than or equal to about 100 $\mu$m.

Starch is a naturally-occurring polysaccharide that can be derived from many different plant sources, including corn, potatoes, tapioca, rice, and wheat. Starch is composed of amylose and amylopectin units. Starch is commercially available from numerous manufacturers such as Anheuser Busch, Starchem, AE Staley Mfg. Co., Matheson, Coleman & Bell, and Henkel Corp. A preferred starch for use in the present invention is pregelatinized starch meeting the requirements of the Official Monograph of the National Formulary. United States Pharmacopeia & National Formulary 26/21 2843 (U.S. Pharmacopeial Convention, Inc.: Rockville, MD 2003).

Preferably, the amount of tadalafil in the solid pharmaceutical dosage form is between about 0.2% and about 20%, between about 2% and about 18%, between about 2.5% and about 10%, or between about 3% and about 8% by weight.

In a preferred embodiment, the starch to tadalafil weight ratio in the solid dosage form is about 5:1 or more, about 6:1 or more, about 10:1 or more, about 12:1 or more, or about 15:1 or more.

In a preferred embodiment, the starch to tadalafil weight ratio in the solid dosage form is about 100:1 or less. In general, and outside the scope of the invention as claimed, the starch to tadalafil weight ratio is between about 4.5: 1 and about 50:1, between about 4.5:1 and about 30:1. According to the invention, the starch to tadalafil weight ratio is between about 10:1 and about 25:1, between about 10:1 and about 20:1, or between about 15:1 and about 20:1.

In one aspect, the pharmaceutical dosage form of the invention displays a high rate of dissolution. As used herein, a "high rate of dissolution" is exemplified by the release of about 65% or more, preferably about 76% or more, of the tadalafil within 40 minutes, when a sample containing 20 mg of tadalafil is tested in an 1000 mL aqueous medium containing 0.14 wt % sodium lauryl sulfate and 6 g/L $NaH_2PO_4$ and having a pH of 4.5 at 37°C, using USP Apparatus II with paddles rotating at a speed of 50 rpm.

Preferably, about 60% or more, more preferably about 68% or more, of the tadalafil is released within 20 minutes, when a sample containing 20 mg of tadalafil is tested in an 1000 mL aqueous medium containing 0.14 wt % sodium lauryl sulfate and 6 g/L $NaH_2PO_4$ and having a pH of 4.5 at 37°C, using USP Apparatus II with paddles rotating at a speed of 50 rpm.

In one aspect, the pharmaceutical dosage form displays similar dissolution rate as Cialis® of the same dosage at the same time point under the same testing condition. Preferably, the ratio of dissolution rate at the same time point under the same testing condition between the dosage form and Cialis® of the same dosage is between 80% and 125%, between 85% and125%, between 90% and125%, or between 95% and 125%. Preferably, the time point is 20 minutes or 40 minutes. The testing condition is as follows: a sample containing 20 mg of tadalafil is tested in an 1000 mL aqueous medium containing 0.14 wt % sodium lauryl sulfate and 6 g/L $NaH_2PO_4$ and having a pH of 4.5 at 37°C, using USP Apparatus II with paddles rotating at a speed of 50 rpm.

In one aspect, pharmaceutical solid dosage form of the invention is bioequivalent to Cialis® of the same dosage. Preferably, the blood concentration of a test dosage form is determined by $C_{max}$ and/or AUC.

Preferably, the $C_{max}$ ratio of the pharmaceutical solid dosage form to Cialis® of the same dosage is between 80% and 125%, between 85% and 125%, or between 90% and 125%. The above $C_{max}$ ratios may be obtained at fed state and/or fasted state.

Preferably, the AUC ratio of the pharmaceutical solid dosage form to Cialis® of the same dosage is between 80% and 125%, between 85% and 125%, between 90% and 125%, or between 95% and 125%. The above AUC ratios may be obtained at fed state and/or fasted state. Optionally, the above AUC ratios are $AUC_{0\text{-}t=71h}$ ratios. Optionally, the above AUC ratios are $AUC_{0\text{-}\infty}$ ratios.

In one aspect, the pharmaceutical solid dosage form of the invention provides a $C_{max(fed)}$ of about 260 ng/ml or more, preferably at least about 300 ng/ml or more, when dosed at 20 mg of tadalafil. Preferably, the $C_{max(fed)}$ is less than about 450ng/ml. One of ordinary skill in the art will appreciate that $C_{max}$ values will vary with the dose administered.

In one aspect, the pharmaceutical solid dosage form of the invention provides an $AUC_{0\text{-}\infty(fed)}$ of about 6000 ng/mg or more, preferably about 6800 ng/mg or more, more preferably about 7200 ng/mg or more, when dosed at 20 mg of tadalafil. Preferably, the $AUC_{0\text{-}\infty(fed)}$ is less than about 9400 ng/mg. One of ordinary skill in the art will appreciate that AUC values will vary with the dose administered.

In one aspect, the pharmaceutical solid dosage form of the invention provides a $C_{max(fast)}$ of about 240 ng/ml or more, preferably at least about 260 ng/ml or more, when dosed at 20 mg of tadalafil. Preferably, the $C_{max(fast)}$ is less than about 400ng/ml.

In one aspect, the pharmaceutical solid dosage form of the invention provides an $AUC_{0\text{-}\infty(fast)}$ of about 6000 ng/mg or more, preferably at least about 6500 ng/mg or more, when dosed at 20 mg of tadalafil. Preferably, the $AUC_{0\text{-}\infty(fed)}$ is less than about 9400 ng/mg.

In a preferred embodiment, the pharmaceutical solid dosage form of the invention further comprises one or more pharmaceutically acceptable excipients selected from the group consisting of disintegrants, surfactants, binders, lubricants, diluents, and glidants. A skilled person in the art, in view of the present disclosure, would be able to choose suitable excipients. Examples of excipients may be found in Handbook of Pharmaceutical Excipients, 4th Ed. 603-604; Pharmaceutical Press: London 2003.

Preferably, the excipient is a binder. The addition of the binder may both further increase the dissolution effect of the starch and improve compressibility. Examples of suitable binders include, but are not limited to, starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose, and polyethylene glycol.

Preferably, the binder is selected from the group consisting of PVP, polyethylene glycol, sugars, invert sugars, collagen, albumin, celluloses in non-aqueous solvents, polypropylene glycol, polyoxyethylene-polypropylene copolymer, polyethylene ester, polyethylene sorbitan ester, poly(ethylene oxide), hydroxypropylmethyl cellulose, hydroxypropyl cellulose, ethyl cellulose, hydroxyethyl cellulose, microcrystalline cellulose, and mixtures thereof. Preferably, the binder is selected from PVP, hydroxypropylmethyl cellulose, hydroxypropyl cellulose, ethyl cellulose, and hydroxyethyl cellulose. More preferably, the binder is PVP. Preferably, the PVP is selected from at least one of PVP with a viscosity K value of about 25 and PVP with a viscosity K value of about 30.

Preferably, the starch to binder weight ratio in the solid dosage form is between about 1:1 and about 9:1. More preferably, the starch to binder weight ratio is between about 3:1 and about 5:1.

In one aspect, the starch to tadalafil weight ratio is between about 4.5:1 and about 50:1, and the starch to binder weight ratio is between about 1:1 and about 9:1. More preferably, the starch to tadalafil weight ratio is between about 10:1 and about 25:1, and the starch to binder weight ratio is between about 3:1 and about 5:1.

In a preferred embodiment of the invention, the dosage forms of the invention further comprise one or more pharmaceutically acceptable excipients. Preferably, the one or more pharmaceutically acceptable excipients are selected from the group consisting of disintegrants, surfactants, binders, lubricants, diluents and glidants. Preferably, the excipient includes a surfactant. Preferably, the surfactant is selected from the group consisting of meglumine, Tris, poloxamer, sodium lauryl sulfate, and mixtures thereof.

In a preferred embodiment of the invention, the excipient includes a lubricant. Examples of suitable lubricants include, but are not limited to, sodium oleate, sodium stearate, magnesium stearate, sodium benzoate, sodium acetate, sodium chloride.

In a preferred embodiment of the invention, the excipient includes a disintegrant. Preferably, the disintegrant is a combination of sodium bicarbonate and tartaric acid.

Optionally, one or more of preservatives, stabilizers, dyes, flavoring agents, antioxidants, and suspending agents may also be incorporated into the pharmaceutical dosage form. Examples of preservatives include, but are not limited to, sodium benzoate, sorbic acid, and esters of p-hydroxybenzoic acid.

According to the invention, the solid dosage form is a compressed solid dosage form. Preferably, the compressed solid dosage form is a tablet. The resiliency of tablets toward impact is quantified in the pharmaceutical industry by the tablet's hardness and friability.

Tablet hardness is a measure of the tablet's propensity to fracture under applied pressure. Devices for measuring hardness are commercially available from a variety of manufacturers such as KRAEMER (UTS) Ltd. Preferably, the compressed solid dosage forms of this invention have a hardness of at least about 5 Strong-Cobb units ("SCU"), as measured by using a KRAEMER (UTS) system. 1.4 SCU = 1 kilopond. Preferably, the hardness is between about 5 and about 22, between about 7 and about 9, or between about 9 and about 22 SCU.

Preferably, the compressed solid dosage forms of this invention have a friability of about 1% or less, about 0.6% or less, about 0.4% or less, or about 0%. The friability of conventional tablets is measured by the percentage weight loss following a typical standard friability test known to one skilled in the art. For example, friability may be measured under standardized conditions according to USP/NF method 1216, by weighing out a certain number of tablets (generally 20 or more), placing them in a rotating PLEXIGLAS drum, lifting them during replicate revolutions by a radial lever, and then dropping them through the diameter of the drum. After replicate revolutions, the tablets are reweighed and the percentage of powder "rubbed off' and of the broken pieces is calculated. A maximum mean weight loss from the three samples of not more than 1.0% is considered acceptable for most products.

One aspect of the description provides a solid pharmaceutical dosage form comprising tadalafil, wherein the tadalafil has a particle size distribution such that $d_{(0.9)}$ is greater than or equal to 40 μm, wherein about 60% or more of the tadalafil is dissolved within 20 minutes when a sample containing 20mg of tadalafil is tested in 1000 mL of aqueous medium containing 0.14 wt % sodium lauryl sulfate and 6 g/L $NaH_2PO_4$ and having a pH of 4.5 at 37°C, using USP Apparatus II with paddles rotating at a speed of 50 rpm. Preferably, about 68% or more of the tadalafil is dissolved within 20 minutes.

One aspect of the description provides a solid pharmaceutical dosage form comprising tadalafil, wherein the tadalafil has a particle size distribution such that $d_{(0.9)}$ is greater than or equal to 40 μm, wherein about 65% or more of the tadalafil is dissolved within 40 minutes when a sample containing 20mg of tadalafil is tested in 1000 mL of aqueous medium containing 0.14 wt % sodium lauryl sulfate and 6 g/L $NaH_2PO_4$ and having a pH of 4.5 at 37°C, using USP Apparatus II with paddles rotating at a speed of 50 rpm. Preferably, about 76% or more of the tadalafil is dissolved within 40 minutes.

One embodiment of the invention provides a process for preparing any of the claimed solid pharmaceutical dosage forms comprising tadalafil, as further defined in the claims.

One embodiment of the invention provides a process for preparing a solid dosage form of tadalafil by using wet granulation method or wet double compression method. The wet double compression method is advantageously used to make compressed solid dosage forms with a high content of a finely divided hydrophilic material such as starch.

Several hydrophilic materials can be used in the manufacturing process. In the processes of the present invention, a relatively high concentration of starch in the composition has marked effect on improving the dissolution rate of the final dosage form. Thus, a preferred hydrophilic excipient is starch.

The process may comprises the following steps:

In the first step, a pharmacologically active compound in particulate form is blended with powdered hydrophilic material, preferably starch, and optionally with a surfactant (e.g., meglumin, Tris, or sodium lauryl sulfate). The materials should be mixed well to produce a homogenous blend. The quantity of the pharmacologically active compound and hydrophilic material to be blended will be determined by taking into consideration the desired ratio of ingredients, potency, and the size of the final compressed dosage form.

In addition to the active compound, the hydrophilic material, and the optional surfactant, other ingredients may also be blended at this stage. Examples include, but are not limited to, lubricants, disintegrants, binders, and diluents. Blending can be performed by any known method and by using any equipment capable of producing a homogeneous powder mixture, such as a V-cone blender, powder mixer, high shear mixer, or fluidized bed granulator.

Optionally, the blend from the first step is compressed into a coherent solid, for example, a slug, ribbon, or sheet. This may be done using conventional dry granulation techniques such as slugging and roller compactions. In a preferred aspect, the coherent solid is in the form of a slug. In another aspect, the coherent solid is in the form of a compacted sheet or ribbon.

Preferably, the coherent solid is comminuted into granules. Preferably, the comminution step comprises passing the coherent solid through a screen. Preferably, this step comprises milling the coherent solid into a milled solid. Comminution may be performed with a mill such as a Fitzpatrick mill or by screening. Preferably, this step further comprises

screening the milled solid so produced.

In the second step, the blend or the granules from the first step are wet-granulated. When the product of the first step is a blend, a granulation liquid is added to the blend to form granules. When the product of the first step is granules, the granules are wetted with a granulation liquid.

Preferably, the granulation liquid is selected from water, a $C_1$-$C_4$ alcohol, and mixtures thereof. More preferably, the granulation liquid is ethanol.

In one aspect, the granules of the second step further comprises one or more pharmaceutically accepted excipients. Examples of the excipients include, but are not limited to disintegrant, binder, lubricant, and glidant. Preferably, the excipient is a binder. Optionally, the binder is dissolved or dispersed in the granulation liquid before the blend or granules from the first step are wetted with the granulation liquid.

Suitable binders include, for example, PVP, polyethylene glycol, sugars, invert sugars, , collagen, albumin, celluloses in non-aqueous solvents, poly(propylene glycol), polyoxyethylene-polypropylene copolymer, polyethylene ester, polyethylene sorbitan ester, poly(ethylene oxide), hydroxypropyl cellulose, ethyl cellulose, hydroxyethyl cellulose, hydroxypropylmethyl cellulose, and microcrystalline cellulose. Preferably, the binder is selected from PVP, hydroxypropylmethyl cellulose, hydroxypropyl cellulose, ethyl cellulose, and hydroxyethyl cellulose. More preferably, the binder is PVP, more preferably PVP selected from at least one of PVP with a viscosity K value of about 25 and PVP with a viscosity K value of or about 30. In a preferred embodiment, the binder is PVP and the granulation liquid is ethanol. Preferably, the concentration of the PVP in the solution is between about 40% to about 80%, more preferably between about 55% to about 65% by weight.

Optionally, additional solid ingredients may be added. Such solid ingredients include, but are not limited to filler, acidifying agent, alkalizing agent, adsorbent, antioxidant, buffering agent, colorant, electrolyte, emulsifying (suspending) agent, flavorant, fragrance, sweetening agent, antiadherent, binder, diluent, excipient, disintegrant, glidant, lubricant, opaquant, and polishing agent. Suitable fillers include, e.g., dibasic calcium phosphate, kaolin, sucrose, mannitol, microcrystalline cellulose, powdered cellulose, precipitated calcium carbonate, sorbitol, starch, lactose, and combinations thereof.

Guidance as to the quantity of liquid to use and the type and quantity of additional ingredients that may be added during this wetting step can be obtained by reference to the known conditions employed in conventional wet granulating processes. Accordingly, enough of the liquid is used so that the granules and any other solid ingredients included in this step will be thoroughly wetted, yet not so much that there is a significant amount of free-flowing liquid remaining after all of the insoluble ingredients have been added.

In the third step, the wet granules from the second step are dried. Drying can be performed using any conventional drying equipment such as a tray dryer or a fluid bed dryer. The drying temperature will depend partly upon the thermolability of the active ingredient.

Optionally, one or more pharmaceutically acceptable excipients are blended with the dried granules. For example, if the granules are to be tabletted, it may be desirable to add a glidant and/or a lubricant before loading the granules into the feed hopper of a tabletting machine.

Optionally, the dried granules from the third step are comminuted. Comminution may be performed with a mill such as a Fitzpatrick mill and/or by screening.

Optionally, the comminuted granules are compressed into a coherent solid, for example, a slug, ribbon, or sheet. This may be done using conventional dry granulation techniques such as slugging and roller compactions. Preferably, the coherent solid is in the form of a slug.

Preferably, the coherent solid is comminuted into granules. Preferably, the comminution step comprises passing the coherent solid through a screen. Preferably, this step comprises milling the coherent solid into a milled solid. Comminution may be performed with a mill such as a Fitzpatrick mill or by screening. Preferably, this step further comprises screening the milled solid so produced.

In a preferred embodiment, the granules from the third step are compressed to form a compressed solid dosage form. Preferably, the compressed solid dosage form is a tablet. Optionally, the tablets are coated. Optionally, the coated or uncoated tablets are packaged in conventional manner with appropriate labeling instructing doctors and patients on the proper use of the tablets.

Preferably, the compressed solid dosage form has a hardness of at least about 5, preferably between about 5 and about 22, between about 7 and about 9, or between about 9 and about 22 Strong-Cobb units, as measured by using a KRAEMER (UTS) system. Preferably, the compressed solid dosage forms of this invention have a friability of about 1% or less, about 0.6% or less, about 0.4% or less, or about 0%, as measured by using the testing method as described above.

In accordance with the present invention, tadalafil may be used in treating a disease or condition by orally administering such drugs in the dosage forms of the invention. Thus, a further embodiment of the invention provides tadalafil for use in a method of treating a disease or condition comprising orally administering to a patient a solid pharmaceutical dosage form of the invention. In a preferred embodiment, the disease is male erectile dysfunction.

One embodiment of the invention provides a solid pharmaceutical dosage form of the invention for use in medicine.

One embodiment of the invention provides the use of a solid pharmaceutical dosage form of the invention in the preparation of a medicament for treating male erectile dysfunction.

The following formulation examples are illustrative only and are not intended to

EXAMPLES

General

Unless otherwise specified, dissolution profiles were generated by testing samples containing 20mg of tadalafil in 1000 mL of an aqueous solution of 0.14% (w/w) sodium lauryl sulfate and 6g/L $NaH_2PO_4$ have a pH of 4.5 at 37°C, using USP Apparatus II with paddles rotating at a speed of 50 rpm.

Unless otherwise specified, the tadalafil used in the examples has a particle size distribution such that $d_{(0.9)}$ is between about 45 and about 58 μm. Particle size distribution was measured by laser diffraction. Instruments for measuring particle size distribution Malvern Instruments Ltd. (U.K.).

Unless otherwise specified, the starch used in the examples is partially pregelatinized maize starch (Starch 1500®, Colorcon, West Point, PA), the PVP used in the examples has a viscosity K value of about 25 and/or about 30.

Tablet hardness was measured by determining lateral breaking strength using a KRAEMER (UTS) system.

Tablet friability was measured by the method describe above.

Example 1 (a-d)

An example composition was prepared from the ingredients listed in Table 1 following the steps as described below. Samples taken from various stages of the process were tested for dissolution rates. The dissolution profiles of Formulation 1 samples are shown below in Table 9 and Figure 1.

Table 1

| Ingredient | Weight (mg/tablet) | Weight Percent |
|---|---|---|
| Part I | | |
| Tadalafil | 20 | 4.1 % |
| Starch | 350 | 71.4 % |
| Granulation solution | | |
| PVP | 120 | 24.5 % |
| Ethanol (95%, USP) | | |

1. Part I ingredients were thoroughly blended (example 1a);
2. The blend was pressed to slugs. (example 1b);
3. The slugs were milled (example 1c);
4. Granulation solution (PVP dissolved in ethanol) was added to the milled slugs;
5. The wet granules obtained from step 4 were dried and milled into dry granules (example 1d).

Comparative Example 2

A tadalafil tablet having conventional fillers (microcrystalline cellulose and lactose) was made by wet granulation. The ingredients in Table 2 were wet granulated and then compressed into tablets weighing 400 mg. The dissolution profile of Formulation 2 is shown below in Table 9 and Figure 1.

Table 2

| Ingredient | Weight (mg/tablet) | Weight Percent |
|---|---|---|
| Part I | | |
| Tadalafil | 20* | 5% |
| Lactose | 20 | 5% |

(continued)

| Ingredient | Weight (mg/tablet) | Weight Percent |
|---|---|---|
| Part I | | |
| Sodium lauryl sulfate | 0.4 | 0.1% |
| Part II | | |
| Microcrystalline cellulose (Avicel , FMC Biopolymer) | 86 | 22 % |
| Colloidal fumed silica (Aerosil®, Degussa, now Evonic Industries) | 2 | 0.5 % |
| Hypromellose | 16 | 4 % |
| Sodium starch glycolate (Primojel®, DMV International) | 20 | 5 % |
| Lactose | 99.6 | 25 % |
| Granulation solution | | |
| Purified water | | |
| Poloxamer | 20 | 5 % |
| Part III | | |
| Avicel® | 86 | 23 % |
| Cross-linked carboxymethylcellulose sodium (Primellose®, DMV International) | 20 | 5 % |
| Part IV | | |
| Sodium stearyl fumarate | 10 | 2.5 % |
| * The actual amount was 26mg, based on an assay purity of 130%. | | |

1. Part I ingredients were co-micronized;
2. Part I and Part II ingredients were thoroughly blended;
3. The blend was granulated by adding the granulation solution (poloxamer dissolved in water), the granules were then dried and milled;
4. Part II ingredients were then blended with the granules from step 2;
5. Part III ingredient was then blended with the granules from step 2 for about 5 minutes;
6. The lubricated granules from step 4 were compressed into tablets.

Example 3 (slugging + wet granulation; Starch:Tadalafil weight ratio = 17.5: 1; Starch:PVP K-30 weight ratio = 3.2:1)

Tadalafil granules were made from the ingredients listed in Table 3 by the slugging and wet granulation method as described below. The dissolution profile of Formulation 3 is shown below in Table 9 and Figure 1.

Table 3

| Ingredient | Weight (mg/tablet) | Weight Percent |
|---|---|---|
| Part I | | |
| Starch 1500® | 350 | 56 % |
| Tadalafil | 20 | 3.2 % |
| Sodium stearyl fumarate | 2 | 0.32% |
| Part II | | |
| PVP K-30 | 110 | 17.8 % |
| Ethanol (95 %, USP) | | |
| Part III | | |
| Sodium bicarbonate | 60 | 9.7 % |
| Tartaric acid | 40 | 6.5 % |
| Aerosil® 200 | 1.5 | 0.24 % |

(continued)

| | | |
|---|---|---|
| Part III | | |
| Avicel® 101 | 30 | 4.9 % |
| Part IV | | |
| Sodium stearyl fumarate | 6 | 0.97 % |

1. Part I ingredients were thoroughly blended and pressed to slugs;
2. The slugs were milled to obtain granules;
3. The granules were wetted with PVP solution in ethanol;
4. The wet granules were dried and milled;
5. Part III ingredients were mixed together with the granules;
6. Part IV ingredient was then blended with the granules for about 5 minutes;
7. The mixture of step 6 was pressed into tablets.

Example 3a (slugging + wet granulation; Starch:Tadalafil weight ratio = 17.5:1; Starch:PVP K-30 weight ratio = 3.2:1)

Tadalafil granules were made from the ingredients listed in Table 3a by the slugging and wet granulation method as described in Example 3. The hardness of the tablet was 7-9 SCU. The friability of the tablet was 0%.

Table 3a

| Ingredient | Weight (mg/tablet) | Weight Percent |
|---|---|---|
| Part I | | |
| Starch 1500® | 350 | 56 % |
| Tadalafil | 20 | 3.2 % |
| Sodium stearyl fumarate | 4 | 0.64 % |
| Part II | | |
| PVP K-30 | 110 | 17.8 % |
| Ethanol (95 %, USP) | | |
| Part III | | |
| Sodium bicarbonate | 60 | 9.7 % |
| Tartaric acid | 40 | 6.5 % |
| Aerosil® 200 | 2 | 0.32 % |
| Avicel® 101 | 30 | 4.9 % |
| Part IV | | |
| Sodium stearyl fumarate | 6 | 0.97 % |

Example 4: (Starch:Tadalafil weight ratio = 12.5:1; Starch:PVP K-30 weight ratio = 2.9:1)

Tadalafil was mixed with starch, pressed to slugs, milled, and wet granulated following the steps as described below. The dissolution profile of Formulation 4 is shown below in Table 9 and Figure 1.

Table 4

| Ingredient | Weight (mg/tablet) | Weight Percent |
|---|---|---|
| Part I | | |
| Tadalafil | 20 | 4.4 % |
| Starch | 250 | 54.9 % |
| Part II | | |
| PVP K-30 | 85 | 18.7 % |
| Ethanol (95%, USP) | | |

(continued)

| | | |
|---|---|---|
| Part III | | |
| Sodium bicarbonate | 60 | 13.2 % |
| Tartaric acid | 40 | 8.9 % |

1. Part I ingredients were thoroughly blended and pressed to slugs;
2. The slugs were milled;
3. The granules were wet granulated with PVP solution in alcohol;
4. The wet granules were dried and milled;
5. Part III ingredients were mixed together with the granules;
6. The granules were pressed into tablets.

Example 5 (Wet granulation before slugging; Starch:Tadalafil weight ratio = 17.5:1; Starch:PVP K-30 weight ratio = 2.9:1)

Tadalafil granules were made from the ingredients listed in Table 5 by wet granulation and slugging method as described below.. The dissolution profile of Formulation 5 is shown below in Table 9 and Figure 1.

Table 5

| Ingredient | Weight (mg/tablet) | Weight Percent |
|---|---|---|
| Part I | | |
| Tadalafil | 20 | 3.3% |
| Starch | 350 | 57.8 % |
| Tris | 15 | 2.5 % |
| Granulation solution | | |
| PVP | 120 | 19.8 % |
| Ethanol (95%, USP) | | |
| Part III | | |
| Sodium bicarbonate | 60 | 9.9 % |
| Tartaric acid | 40 | 6.6% |

1. Part I ingredients were thoroughly blended;
2. The granulation solution (PVP dissolved in ethanol) was added to the blend to form granules;
3. The granules were dried and milled by mortar and pestle and sifted through a size 30 mesh;
4. The granules were pressed to slugs and milled;
5. Part III ingredients were then added and mixed thoroughly;
6. The granulate was pressed into tablets.

Example 6 (Wet granulation without slugging; Starch:Tadalafil weight ratio = 17.5:1; Starch:PVP K-30 weight ratio = 2.9:1)

Tadalafil granules are made from the ingredients listed in Table 6 by wet granulation method as described below.

Table 6

| Ingredient | Weight (mg/tablet) | Weight Percent |
|---|---|---|
| Part I | | |
| Tadalafil | 20 | 3.3 % |
| Starch | 350 | 57.8 % |
| Tris | 15 | 2.5 % |
| Granulation solution | | |
| PVP | 120 | 19.8 % |
| Alcohol | | |

(continued)

| | | |
|---|---|---|
| Part III | | |
| Sodium bicarbonate | 60 | 9.9 % |
| Tartaric acid | 40 | 6.6 % |

1. Part I ingredients are thoroughly blended;
2. Granulation solution (PVP dissolved in alcohol) is added to the blend;
3. The granules are dried and milled by mortar and pestle, and then sifted through a size 30 mesh;
4. Part III ingredients are then added and mixed thoroughly;
5. The granules are pressed into tablets.

Example 7 (Without slugging; Starch:Tadalafil weight ratio = 5:1; Starch:PVP K-30 weight ratio = 5:1)

Tadalafil tablets containing starch were made by wet granulation without slugging as described below. The dissolution profile of Formulation 7 is shown below in Table 9 and Figure 1.

Table 7

| Ingredient | Weight (mg/tablet) | Weight Percent |
|---|---|---|
| Part I | | |
| Starch 1500® | 100 | 17.9 % |
| Tadalafil | 20 | 3.6% |
| Sodium lauryl sulfate | 20 | 3.6 |
| Part II | | |
| Aerosil® | 2.0 | 0.36 % |
| Sodium starch glycolate (Primojel®) | 20 | 3.6 % |
| Hypromellose (Methocel® E5, Dow Chemical) | 16 | 2.9% |
| Meglumine | 40 | 7.1 % |
| Lactose monohydrate | 60 | 10.7 % |
| Granulation solution I | | |
| Poloxamer 188 (Lutrol® F-68, BASF) | 18 | 3.6% |
| Ethanol (95%, USP) | | |
| Granulation solution II | | |
| PVP K-30 | 20 | 3.6 % |
| Ethanol (95%, USP) | | |
| Part III | | |
| Lactose, spray dried | 60 | 10.7 % |
| Primellose® | 20 | 3.6% |
| Sodium bicarbonate | 90 | 16.1 % |
| Tartaric acid | 60 | 10.7 % |
| Part IV | | |
| Sodium stearyl fumarate | 12 | 2.1 % |

1. Part I ingredients were thoroughly blended;
2. Part II ingredients were added and blended with the part I blend;
3. The granulation solution I was added, followed by addition of granulation solution II, followed by mixing;
4. After wetting, the granules were dried and milled;
5. Part III ingredients were added and blended well;
6. Part IV ingredient were added and mixed.
7. The final granules were pressed into tablets.

Comparative Example 8 (based on Formulation 5 of U.S. 2006/0099252; Starch:Tadalafil ratio = 4.4:1)

Tadalafil granules were made from the ingredients listed in Table 8 by the slugging and wet granulation method as described below. This is an example using 75% starch and a starch to tadalafil weight ratio of 4.4:1. The dissolution profile of Formulation 8 is shown below in Table 9 and Figure 1.

Table 8

| Ingredient | Weight (mg/tablet) | Weight Percent |
|---|---|---|
| Part I | | |
| Starch 1500® | 88 | 75 % |
| Tadalafil | 20 | 17.1 % |
| Magnesium stearate | 0.3 | 0.26% |
| Part II | | |
| PVP K-30 | 3 | 2.6% |
| Ethanol (95%, USP) | | |
| Part III | | |
| Microcrystalline cellulose | 4.8 | 4.1 % |
| Aerosil® | 0.3 | 0.26% |
| Part IV | | |
| Magnesium stearate | 0.7 | 0.6 % |

1. Part I ingredients were thoroughly blended and pressed to slugs;
2. The slugs were milled;
3. The granules were wet granulated with PVP solution in alcohol;
4. The wet granules were dried and milled;
5. Part III ingredients were mixed together with the granules;
6. The mixture of step 5 was pressed to tablets.

Dissolution Testing Results

Table 9: Dissolution Profile of example formulations and reference product (% dissolution)

| time (min) | Ex 1(a) | Ex (b) | Ex 1(c) | Ex 1(d) | Ex 2 | Ex 3 | Ex 4 | Ex 5 | Ex 7 | Ex 8 | Cialis® |
|---|---|---|---|---|---|---|---|---|---|---|---|
| 0 | 0 | 0 | 0 | 0.0 | 0 | 0 | 0 | 0 | 0 | 0 | 0 |
| 5 | 10.7 | 10.2 | 39.9 | 50.9 | 20.4 | 36.2 | 50.7 | 57.7 | 39 | 18.3 | 44.4 |
| 10 | 19.7 | 20 | 52.7 | 68.3 | 31 | 63 | 67.6 | 70.6 | 54.9 | 32.5 | 65.1 |
| 20 | 29.8 | 33.7 | 61.4 | 69.6 | 43.5 | 73.5 | 70.5 | 68 | 64.9 | 42.9 | 77.6 |
| 30 | 37.4 | 45.3 | 67.6 | 78.6 | 47.9 | 68.1 | 65.9 | 76.4 | 61.5 | 51.2 | 71.0 |
| 40 | 43.7 | 45.7 | 69.5 | 71.6 | 52.9 | 79.5 | 76 | 69.8 | 68.3 | 55.4 | 84.9 |
| 60 | 48.5 | 55.3 | 72.8 | 78.8 | 58.3 | 82.9 | 75.4 | 77.6 | 74.1 | 58.4 | 85.3 |

Example 9: Pharmacokinetic Study

An open-label, single-dose, randomized, three-period, three sequence, three-treatment, crossover study was conducted with 12 subjects. The study was designed to compare the relative bioavailability of two tadalafil formulations (Examples 3a and 7). The reference product was Cialis®, 20 mg. The pharmacokinetic parameters of tadalafil under fasted and fed states are listed in Table 10.

Table 10: Pharmacokinetic study results of example formulations and reference product

| | Example 3a | Example 7 | Cialis® Lot No. A149562 |
|---|---|---|---|
| $C_{max(fast)}$ (ng/ml) | 274 | 259 | 308 |
| $C_{max(fast)}$ ratio | 89.0% | 84.1% | - |
| $AUC_{0-t(fast)}$ (ng/mg) | 5938 | 5941 | 6677 |
| $AUC_{0-t(fast)}$ ratio | 88.9% | 89.0% | - |
| $AUC_{0-\infty(fast)}$ (ng/mg) | 6539 | 6631 | 7409 |
| $AUC_{0-\infty(fast)}$ ratio | 88.3% | 89.5% | - |
| $C_{max(fed)}$ (ng/ml) | 306 | 260 | 340 |
| $C_{max(fed)}$ ratio | 90.0% | 76.5% | - |
| $AUC_{0-t(fed)}$ (ng/mg) | 6696 | 6364 | 7020 |
| $AUC_{0-t(fed)}$ ratio | 95.4% | 90.7% | - |
| $AUC_{0-\infty(fed)}$ (ng/mg) | 7228 | 6881 | 7467 |
| $AUC_{0-\infty(fed)}$ ratio | 96.8% | 92.2% | - |

All $AUC_{0-t}$ values were taken at t = 71 hours.

All values of $C_{max}$ and AUC presented as geometric means of multiple subjects.

## Claims

1. A compressed solid pharmaceutical dosage form comprising tadalafil, a pregelatinised starch, and a binder, wherein the tadalafil has a particle size distribution such that $d_{(0.9)}$, as measured by laser diffraction, is greater than or equal to 40 μm, wherein the weight ratio of starch to tadalafil is 5:1 or more, 6:1 or more, 10:1 or more, 12:1 or more, or 15:1 or more, and is 100:1 to less.

2. The dosage form of Claim 1, wherein the weight ratio of starch to tadalafil is between 10:1 and 25:1, between 10:1 and 20:1, or between 15:1 and 20:1.

3. The dosage form of any preceding claim, wherein the amount of tadalafil in the dosage form is between 0.2% and 20% by weight, optionally between 2% and 18% by weight, between 2.5% and 10% by weight, or between 3% and 8% by weight.

4. The dosage form of any preceding claim, wherein 60% or more, preferably 68% or more, of the tadalafil is dissolved within 20 minutes when a sample containing 20mg of tadalafil is tested in 1000 mL of aqueous medium containing 0.14 wt % sodium lauryl sulfate and 6 g/L $NaH_2PO_4$ and having a pH of 4.5 at 37°C, using USP Apparatus II with paddles rotating at a speed of 50 rpm.

5. The dosage form of any preceding claim, wherein 65% or more, preferably 76% or more, of the tadalafil is dissolved within 40 minutes when a sample containing 20mg of tadalafil is tested in 1000 mL of aqueous medium containing

0.14 wt % sodium lauryl sulfate and 6 g/L $NaH_2PO_4$ and having a pH of 4.5 at 37°C, using USP Apparatus II with paddles rotating at a speed of 50 rpm.

**6.** The dosage form of any preceding claim, wherein the tadalafil has a particle size distribution such that $d_{(0.9)}$ is greater than or equal to 50 μm, optionally greater than or equal to 60 μm, or greater than or equal to 70 μm.

**7.** The dosage form of any preceding claim, wherein the tadalafil has a particle size distribution such that $d_{(0.9)}$ is less than or equal to 150 μm, and preferably less than or equal to 100 μm.

**8.** The dosage form of any preceding claim, further comprising one or more pharmaceutically acceptable excipients selected from the group consisting of disintegrants, surfactants, binders, lubricants, diluents, and glidants.

**9.** The dosage form of any preceding claim, wherein the binder is selected from the group consisting of PVP, hydroxypropylmethyl cellulose, hydroxypropyl cellulose, ethyl cellulose, hydroxyethyl cellulose, and mixtures thereof, more preferably wherein the binder is PVP, and most preferably wherein the binder is PVP which is selected from at least one of PVP with a viscosity K value of 25 and PVP with a viscosity K value of 30.

**10.** The dosage form of claim 9, wherein the starch to binder weight ratio in the solid dosage form is from 1:1 to 9:1, preferably from 3:1 to 5:1.

**11.** The dosage form of any of Claims 8 to 10, further comprising a surfactant, preferably wherein the surfactant is selected from the group consisting of meglumine, tris(hydroxymethyl)amine, sodium lauryl sulfate, and mixtures thereof.

**12.** The dosage form of any of Claims 8 to 11, further comprising a lubricant, preferably wherein the lubricant is selected from the group consisting of sodium oleate, sodium stearate, magnesium stearate, sodium benzoate, sodium acetate, sodium chloride, and mixtures thereof.

**13.** The dosage form of any of Claims 8 to 12, further comprising a disintegrant, preferably wherein the disintegrant is a combination of sodium bicarbonate and tartaric acid.

**14.** The dosage form of any preceding claim, further comprising one or more of preservatives, stabilizers, dyes, flavoring agents, antioxidants, and suspending agents.

**15.** The dosage form of any preceding claim, wherein the compressed solid dosage form is a tablet.

**16.** The compressed solid dosage form of any preceding claim, wherein the hardness of the compressed solid dosage form is 35.02 N (5 Strong-Cobb units) or more, 35.02 to 154.10 N (5 to 22 Strong-Cobb units), 49.03 to 63.04 N (7 to 9 Strong-Cobb units), or 63.04 to 154.10 N (9 to 22 Strong-Cobb units).

**17.** The compressed solid dosage form of any preceding claim , wherein the friability of the compressed solid dosage form is 1% or less, preferably 0.6% or less, more preferably 0.4% or less, and most preferably 0%.

**18.** A process for preparing the solid dosage form of any preceding claim, comprising using wet double compression method, or wet granulation method.

**19.** A process for preparing the solid dosage form of any of Claims 1 to 17, comprising:

a) blending particulate tadalafil with starch;
b) wet-granulating the product of step a) with a granulating liquid; and
c) drying the product of step b) to form dried granules.

**20.** The process of Claim 19, wherein the granulation liquid further comprises PVP, wherein the concentration of PVP in solution is 40% to 80% by weight, and preferably 55% to 65% by weight.

**21.** The process of any of Claims 18 to 20, wherein the granules from the last step are compressed to form a compressed solid dosage form.

**22.** The solid pharmaceutical dosage form of any of Claims 1 to 17 for use in medicine.

**23.** The solid pharmaceutical dosage form of any of Claims 1 to 17 for use in treating male erectile dysfunction.

**24.** The use of the solid pharmaceutical dosage form of any of Claims 1 to 17 in the preparation of a medicament for treating male erectile dysfunction.

**Patentansprüche**

**1.** Komprimierte feste pharmazeutische Dosierungsform, umfassend Tadalafil, eine vorverkleisterte Stärke und ein Bindemittel, wobei das Tadalafil eine derartige Partikelgrößeverteilung aufweist, dass der mittels Laserbeugung bestimmte d(0,9)-Wert größer oder gleich 40 μm ist, wobei das Gewichtsverhältnis der Stärke zum Tadalafil 5:1 oder höher, 6:1 oder höher, 10:1 oder höher, 12:1 oder höher oder 15:1 oder höher, und höchstens 100:1 ist.

**2.** Dosierungsform nach Anspruch 1, wobei das Gewichtsverhältnis der Stärke zum Tadalafil zwischen 10:1 und 25: 1, zwischen 10:1 und 20:1 oder zwischen 15:1 und 20: 1 liegt.

**3.** Dosierungsform nach einem der vorstehenden Ansprüche, wobei die Tadalafilmenge in der Dosierungsform zwischen 0,2 und 20 Gew. %, wahlweise zwischen 2 und 18 Gew. %, zwischen 2,5 und 10 Gew. % oder zwischen 3 und 8 Gew. % liegt.

**4.** Dosierungsform nach einem der vorstehenden Ansprüche, wobei 60 % oder mehr, vorzugsweise 68 % oder mehr des Tadalafils binnen 20 Minuten aufgelöst wird, wenn eine Probe, die 20 mg Tadalafil enthält, in 1000 mL eines wässrigen Mediums, das 0,14 Gew. % Natriumlaurylsulfat und 6 g/L NaH2PO4 enthält und bei 37 °C einen pH-Wert von 4,5 aufweist, unter Einsatz von USP Apparatus II bei einer Flügeldrehzahl von 50 getestet wird.

**5.** Dosierungsform nach einem der vorstehenden Ansprüche, wobei 65 % oder mehr, vorzugsweise 76 % oder mehr des Tadalafils binnen 40 Minuten aufgelöst wird, wenn eine Probe, die 20 mg Tadalafil enthält, in 1000 mL eines wässrigen Mediums, das 0,14 Gew. % Natriumlaurylsulfat und 6 g/L NaH2PO4 enthält und bei 37 °C einen pH-Wert von 4,5 aufweist, unter Einsatz von USP Apparatus II bei einer Flügeldrehzahl von 50 getestet wird.

**6.** Dosierungsform nach einem der vorstehenden Ansprüche, wobei das Tadalafil eine derartige Partikelgrößeverteilung aufweist, dass der d(0,9)-Wert größer oder gleich 50 μm, wahlweise größer oder gleich 60 μm oder größer oder gleich 70 μm ist.

**7.** Dosierungsform nach einem der vorstehenden Ansprüche, wobei das Tadalafil eine derartige Partikelgrößeverteilung aufweist, dass der d(0,9)-Wert größer oder gleich 150 μm, vorzugsweise kleiner oder gleich 100 μm ist.

**8.** Dosierungsform nach einem der vorstehenden Ansprüche, ferner umfassend einen oder mehrere pharmazeutisch annehmbare Hilfsstoffe, die aus der Gruppe der Sprengmittel, Tenside, Bindemittel, Gleitmittel, Verdünnungsmittel und Gleitstoff gewählt wird.

**9.** Dosierungsform nach einem der vorstehenden Ansprüche, wobei das Bindemittel aus der Gruppe von PVP, Hydroxypropylmethylcellulose, Hydroxypropylcellulose, Ethylcellulose, Hydroxyethylcellulose und deren Gemischen gewählt wird, weiter bevorzugt wobei das Bindemittel PVP ist, und am meisten bevorzugt wobei das Bindemittel PVP ist, das mindestens einem von PVP mit einem Viskositätswert K von 25 und PVP mit einem Viskositätswert K 30 gewählt ist.

**10.** Dosierungsform nach Anspruch 9, wobei das Gewichtsverhältnis der Stärke zum Bindemittel in der festen Dosierungsform zwischen 1:1 und 9:1, vorzugsweise zwischen 3:1 und 5:1 liegt.

**11.** Dosierungsform nach einem der Ansprüche 8 bis 10, ferner umfassend ein Tensid, vorzugsweise wobei das Tensid aus der Gruppe von Meglumin, tris(Hydroxymethyl)amin, Natriumlaurylsulfat und deren Gemischen gewählt wird.

**12.** Dosierungsform nach einem der Ansprüche 8 bis 11, ferner umfassend ein Gleitmittel, vorzugsweise wobei das Gleitmittel aus der Gruppe von Natriumoleat, Natriumstearat, Magnesiumstearat, Natriumbenzoat, Natriumacetat, Natriumchlorid und deren Gemischen gewählt wird.

**13.** Dosierungsform nach einem der Ansprüche 8 bis 12, ferner umfassend ein Sprengmittel, vorzugsweise wobei das Sprengmittel eine Kombination aus Natriumhydrogencarbonat und Weinsäure ist.

**14.** Dosierungsform nach einem der vorstehenden Ansprüche, ferner umfassend eines oder mehrere von Konservierungsstoffen, Stabilisatoren, Farbstoffen, Aromastoffen, Antioxidantien und Suspensionsmitteln.

**15.** Dosierungsform nach einem der vorstehenden Ansprüche, wobei die komprimierte feste Dosierungsform eine Tablette ist.

**16.** Komprimierte feste Dosierungsform nach einem der vorstehenden Ansprüche, wobei die Härte der komprimierten Dosierungsform 35,02 N (5 Strong-Cobb-Einheiten) oder höher, 35,02 bis 154,10 N (5 bis 22 Strong-Cobb-Einheiten), 49,03 bis 63,04 N (7 bis 9 Strong-Cobb-Einheiten) oder 63,04 bis 154,10 N (9 bis 22 Strong-Cobb-Einheiten) ist.

**17.** Komprimierte feste Dosierungsform nach einem der vorstehenden Ansprüche, wobei die Zerreibbarkeit der komprimierten festen Dosierungsform 1 % oder weniger, vorzugsweise 0,6 % oder weniger, noch weiter bevorzugt 0,4 % oder weniger und am liebsten 0 % ist.

**18.** Verfahren zur Herstellung der festen Dosierungsform nach einem der vorstehenden Ansprüche, umfassend die Verwendung des nassen Doppelkompressionsverfahrens bzw. des nassen Granulierungsverfahrens.

**19.** Verfahren zur Herstellung der festen Dosierungsform nach einem der Ansprüche 1 bis 17, umfassend:

a) Vermischen des partikelförmigen Tadalafils mit Stärke;
b) nasses Granulieren des Produktes des Schritts a) mit einer Granulierflüssigkeit; und
c) Trocknen des Produktes des Schritts b), um getrocknete Körnchen zu bilden.

**20.** Verfahren nach Anspruch 19, wobei die Granulierflüssigkeit ferner PVP umfasst, wobei die PVP-Konzentration in der Lösung 40 bis 80 Gew. % und vorzugsweise 55 bis 65 Gew. % ist.

**21.** Verfahren nach einem der Ansprüche 18 bis 20, wobei die Körnchen aus dem letzten Schritt komprimiert werden, um eine komprimierte feste Dosierungsform zu ergeben.

**22.** Feste pharmazeutische Dosierungsform nach einem der Ansprüche 1 bis 17 zum medizinischen Gebrauch.

**23.** Feste pharmazeutische Dosierungsform nach einem der Ansprüche 1 bis 17 zur Behandlung der Erektionsstörung beim Mann.

**24.** Verwendung der festen pharmazeutischen Dosierungsform nach einem der Ansprüche 1 bis 17 zur Herstellung eines Medikaments zur Behandlung der Erektionsstörung beim Mann.

**Revendications**

**1.** Forme posologique pharmaceutique solide comprimée comprenant du tadalafil, un amidon prégélatinisé et un liant, dans laquelle le tadalafil a une distribution de la dimension des particules telle que $d_{(0,9)}$, telle que mesurée par diffraction laser, est supérieure ou égale à 40 $\mu$m, le rapport en poids de l'amidon au tadalafil étant de 5:1 ou plus, de 6:1 ou plus, de 10:1 ou plus, de 12:1 ou plus ou de 15:1 ou plus, et étant de 100:1 à moins.

**2.** Forme posologique selon la revendication 1, dans laquelle le rapport en poids de l'amidon au tadalafil est entre 10: 1 et 25:1, entre 10:1 et 20:1 ou entre 15:1 et 20:1.

**3.** Forme posologique selon l'une quelconque des revendications précédentes, dans laquelle la quantité de tadalafil dans la forme posologique est entre 0,2 % et 20 % en poids, facultativement entre 2 % et 18 % en poids, entre 2,5 % et 10 % en poids ou entre 3 % et 8 % en poids.

**4.** Forme posologique selon l'une quelconque des revendications précédentes, dans laquelle 60 % ou plus, de préférence 68 % ou plus, du tadalafil sont dissous en l'espace de 20 minutes lorsqu'un échantillon contenant 20 mg de tadalafil est testé dans 1 000 mL de milieu aqueux contenant 0,14 % en poids de lauryl sulfate de sodium et 6 g/L

de $NaH_2PO_4$ et ayant un pH de 4,5 à 37°C, à l'aide de l'USP Apparatus II ayant des pales tournant à une vitesse de 50 tpm.

**5.** Forme posologique selon l'une quelconque des revendications précédentes, dans laquelle 65 % ou plus, de préférence 76 % ou plus, du tadalafil sont dissous en l'espace de 40 minutes lorsqu'un échantillon contenant 20 mg de tadalafil est testé dans 1 000 mL de milieu aqueux contenant 0,14 % en poids de lauryl sulfate de sodium et 6 g/L de $NaH_2PO_4$ et ayant un pH de 4,5 à 37°C, à l'aide de l'USP Apparatus II ayant des pales tournant à une vitesse de 50 tpm.

**6.** Forme posologique selon l'une quelconque des revendications précédentes, dans laquelle le tadalafil a une distribution de la dimension des particules telle que $d_{(0,9)}$ est supérieure ou égale à 50 μm, facultativement supérieure ou égale à 60 μm, ou supérieure ou égale à 70 μm.

**7.** Forme posologique selon l'une quelconque des revendications précédentes, dans laquelle le tadalafil a une distribution de la dimension des particules telle que $d_{(0,9)}$ est inférieure ou égale à 150 μm et de préférence inférieure ou égale à 100 μm.

**8.** Forme posologique selon l'une quelconque des revendications précédentes, comprenant en outre un ou plusieurs excipients pharmaceutiquement acceptables choisis dans le groupe consistant en les agents désintégrants, les agents tensioactifs, les liants, les lubrifiants, les diluants et les agents de glissement.

**9.** Forme posologique selon l'une quelconque des revendications précédentes, dans laquelle le liant est choisi dans le groupe consistant en la PVP, l'hydroxypropylméthyl cellulose, l'hydroxypropyl cellulose, l'éthyl cellulose, l'hydroxyéthyl cellulose et leurs mélanges, de façon davantage préférée dans laquelle le liant est la PVP, et de la façon que l'on préfère le plus, dans laquelle le liant est la PVP qui est choisi parmi au moins l'un de la PVP ayant une valeur K de viscosité de 25 et la PVP ayant une valeur K de viscosité de 30.

**10.** Forme posologique selon la revendication 9, dans laquelle le rapport en poids amidon à liant dans la forme posologique solide est de 1:1 à 9:1, de préférence de 3:1 à 5:1.

**11.** Forme posologique selon l'une quelconque des revendications 8 à 10, comprenant en outre un agent tensio-actif, de préférence dans laquelle l'agent tensio-actif est choisi dans le groupe consistant en la méglumine, la tris(hydroxyméthyl)amine, le lauryl sulfate de sodium et leurs mélanges.

**12.** Forme posologique selon l'une quelconque des revendications 8 à 11, comprenant en outre un lubrifiant, de préférence dans laquelle le lubrifiant est choisi dans le groupe consistant en l'oléate de sodium, le stéarate de sodium, le stéarate de magnésium, le benzoate de sodium, l'acétate de sodium, le chlorure de sodium et leurs mélanges.

**13.** Forme posologique selon l'une quelconque des revendications 8 à 12, comprenant en outre un agent désintégrant, de préférence dans laquelle l'agent désintégrant est une combinaison de bicarbonate de sodium et d'acide tartrique.

**14.** Forme posologique selon l'une quelconque des revendications précédentes, comprenant en outre l'un ou plusieurs parmi les conservateurs, les agents stabilisants, les colorants, les agents aromatisants, les anti-oxydants et les agents de suspension.

**15.** Forme posologique selon l'une quelconque des revendications précédentes, dans laquelle la forme posologique solide comprimée est un comprimé.

**16.** Forme posologique solide comprimée selon l'une quelconque des revendications précédentes, dans laquelle la dureté de la forme posologique solide comprimée est de 35,02 N (5 unités Strong-Cobb) ou plus, 35,02 à 154,10 N (5 à 22 unités Strong-Cobb), 49,03 à 63,04 N (7 à 9 unités Strong-Cobb) ou 63,04 à 154,10 N (9 à 22 unités Strong-Cobb).

**17.** Forme posologique solide comprimée selon l'une quelconque des revendications précédentes, dans laquelle la friabilité de la forme posologique solide comprimée est de 1 % ou moins, de préférence de 0,6 % ou moins, de façon davantage préférée de 0,4 % ou moins et de la façon que l'on préfère le plus de 0 %.

**18.** Procédé de préparation de la forme posologique solide selon l'une quelconque des revendications précédentes,

comprenant l'utilisation d'une méthode à double compression par voie humide ou d'une méthode de granulation par voie humide.

**19.** Procédé de préparation de la forme posologique solide selon l'une quelconque des revendications 1 à 17, comprenant :

a) le mélange du tadalafil particulaire avec de l'amidon ;
b) la granulation par voie humide du produit de l'étape a) avec un liquide de granulation ; et
c) le séchage du produit de l'étape b) pour former des granulés séchés.

**20.** Procédé selon la revendication 19, dans lequel le liquide de granulation comprend en outre de la PVP, la concentration de PVP en solution étant de 40 % à 80 % en poids et de préférence de 55 % à 65 % en poids.

**21.** Procédé selon l'une quelconque des revendications 18 à 20, dans lequel les granulés provenant de la dernière étape sont comprimés pour former une forme posologique solide comprimée.

**22.** Forme posologique pharmaceutique solide selon l'une quelconque des revendications 1 à 17, destinée à être utilisée en médecine.

**23.** Forme posologique pharmaceutique solide selon l'une quelconque des revendications 1 à 17, destinée à être utilisé dans le traitement d'un trouble de l'érection chez l'homme.

**24.** Utilisation de la forme posologique pharmaceutique solide selon l'une quelconque des revendications 1 à 17 dans la préparation d'un médicament pour le traitement d'un trouble de l'érection chez l'homme.

**FIGURE 1**

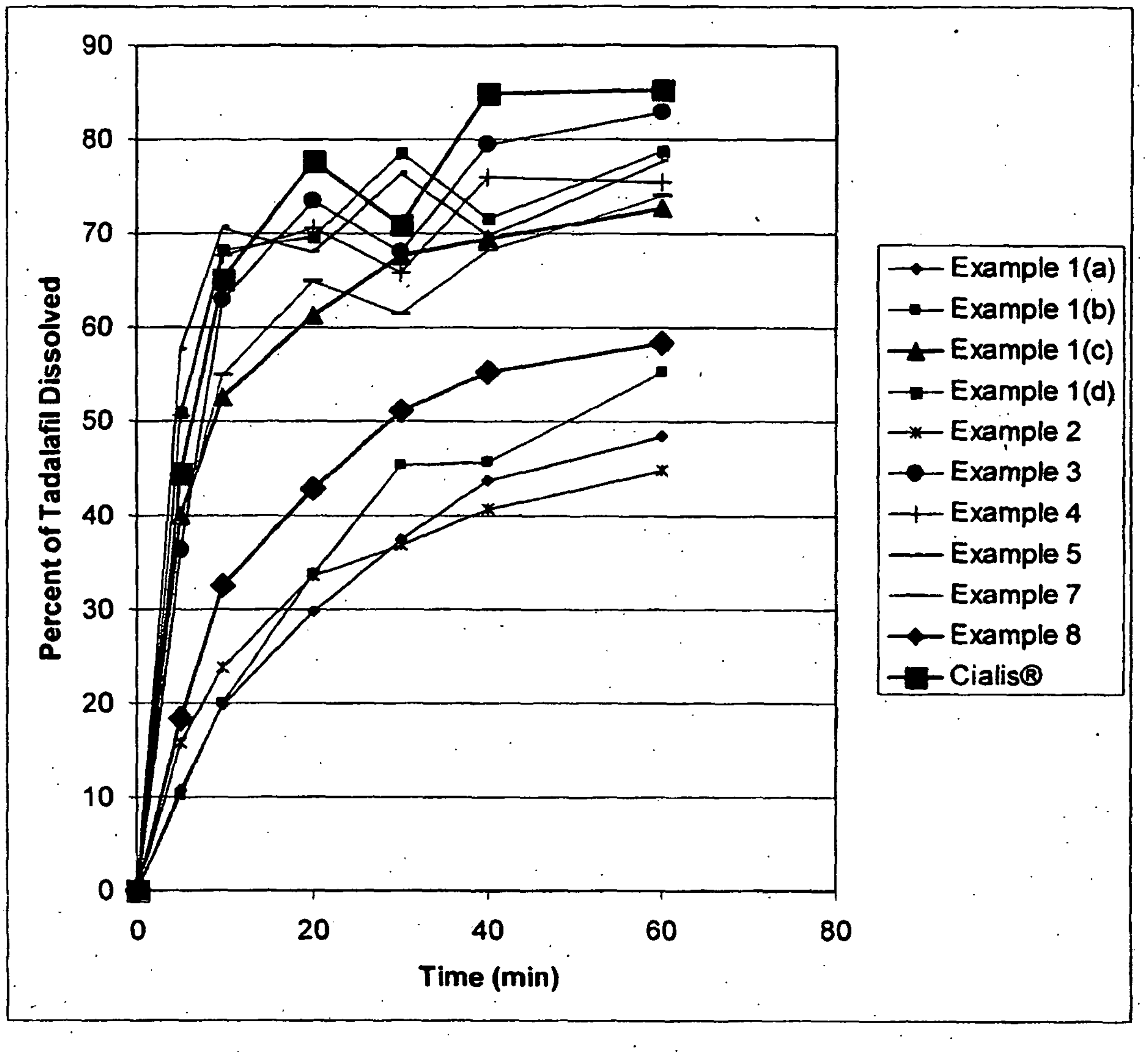
Percent of Tadalafil Dissolved
0
10
20
30
40
50
60
70
80
90
0
20
40
60
80
Time (min)
Example 1(a)
Example 1(b)
Example 1(c)
Example 1(d)
Example 2
Example 3
Example 4
Example 5
Example 7
Example 8
Cialis®

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6841167 B
- US 5985326 A
- US 6821975 B
- US 20060099252 A
- WO 2005000296 A
- US 20080009502 A
- US 2006099252 A


### Non-patent literature cited in the description


- **REMINGTON.** The Science and Practice of Pharmacy. Lippincott Williams & Wilkins, 2000, 656, 657
- **LEVY, G. et al.** *J. Pharm. Sci.,* 1963, vol. 52, 1047
- **FINHOLT, P.** *Medd Norsk Farm. Selsk.,* 1966, vol. 28, 238
- Handbook of Pharmaceutical Excipients. Pharmaceutical Press, 2003, 603-604
- United States Pharmacopeia & National Formulary. U.S. Pharmacopeial Convention, Inc, 2003
- Handbook of Pharmaceutical Excipients. Pharmaceutical Press, 2003